# EUROPEAN PATENT SPECIFICATION

(11) **EP 0 335 770 B1**
(45) Date of publication and mention of the grant of the patent: **15.01.1997**
(21) Application number: 89400773.1
(22) Date of filing: 20.03.1989
(51) Int. Cl.: C07F 9/547, A61K 31/675, C07H 19/10, C07H 19/20, A61K 31/70, C07F 9/40

(54) **Novel fluorophosphonate nucleotide derivatives**
Derivate von Fluorphosphonat-Nukleotiden
Dérivés de nucléotides de fluorophosphonates

(30) Priority: 01.04.1988 EP 88400806
(43) Date of publication of application: 04.10.1989
(73) Proprietor: MERRELL PHARMACEUTICALS INC., Cincinnati, Ohio 45215-6300 (US)
(72) Inventor: Casara, Patrick, F-67117 Ittenheim (FR); Jund, Karin, F-67000 Strasbourg (FR)
(74) Representative: Gillard, Marie-Louise

(56) References cited:
- EP-A- 0 206 459
- CHEMICAL ABSTRACTS, vol. 93, no. 11, September 1980, page 778, abstract no. 114937m, Columbus, Ohio, US;

## Description

This invention relates to fluoromethylphosphonate derivatives of certain nucleosides, to methods for their preparation and to their use as antiviral and antitumoral agents.

More specifically this invention relates to novel fluoromethylphosphonate derivatives of the formula 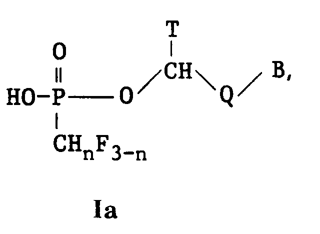 or pharmaceutically acceptable salts thereof, wherein n is an integer zero, one or two, and in which B is a base of the following structural formula 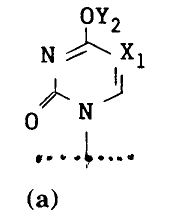 wherein
X₁ is N or CY₁ with Y₁ being H, CH₃, C₂H₅, I, F, Cl, Br, NHR, SR, -C≡CH, -CH=CH₂, -CH=CHBr, -CH₂CH₂Cl, -CH₂CH₂F, or -CH₂C₆H₄OCH₂C₆H₅ (meta),
with
R being H, CH₃, C₂H₅, CH₂C≡CH, CH₂CH=CH₂ or CH₂CN,
Y₂ is H or C₂H₅, and
Q is a cyclic moiety of the formula 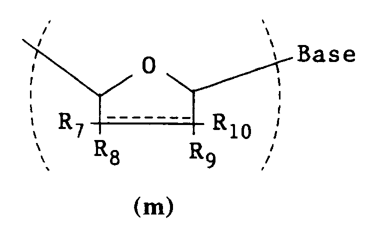
with
R₇ being H, F, Cl
R₈ being H, OH, F, N₃, NH₂, Cl
R₉ being H, OH, F, N₃, NH₂, Cl
R₁₀ being H, F, Cl
T is H, and the dotted line within the structure of formula (m) represents an optional double bond, in which case R₈ and R₉ are omitted and R₇ and R₁₀ are H,

The structural drawings defining the compounds of Formula I do not indicate their stereochemistry and, as it is believed that all configurations would be useful, the stereochemistry of the compounds is not to be construed as a limitation. It is to be appreciated that the depicted structural formulae of the bases represent, where applicable, one of the tautomeric forms. It is to be understood, however, that all of the tautomeric forms are included within the scope of this invention.

In practice, it is preferred to employ bases having a pyrimidine core structure nucleus (including their lactam, lactim and/or tautomeric forms). Preferred pyrimidine type bases are uracil, thymine, 5-halogenated (F, Br or I) or 5-carboxy uracils, Otherwise stated, preferred compounds of this invention are those which contain such nucleosidic moieties as, uridine, deoxyuridine, thymidine,

The term "pharmaceutically acceptable salts" is well known and universally accepted and are those salts useful in the chemotherapy of warm blooded animals. Salts which are especially convenient for therapeutic use are salts of pharmaceutically acceptable organic acids such as lactic, acetic, maleic or p-toluenesulfonic acids as well as salts of pharmaceutically acceptable mineral acids such as hydrochloric, hydrobromic or sulfuric acids.

Representative of the most preferred moieties of the depicted bases of formula (a) for defining compounds of formula Ia are:
5-fluorouracil
5-methyluracil
5-bromouracil
5-iodouracil
5-chlorouracil
5-(2-bromovinyl)uracil
5-hydroxymethyluracil.

The preparation of the desired final compounds of this invention wherein the partial structure is represented by Formula Ia may readily be effected by the coupling of a fluoromethyl phosphonate salt (3) with the appropriate nucleoside (4) by interaction at about 20°C to 40°C for one to three days in the presence of three to five equivalents of dicyclohexylcarbodiimide (DCC) under anhydrous conditions, preferably in an inert atmosphere (nitrogen or argon being preferred). After the reaction is completed it is quenched with water and the N,N'-dicyclohexylurea is filtered, the filtrate washed with diethyl ether and from the aqueous phase the products are isolated and purified using recrystallization or high performance liquid chromatography (HPLC) techniques. With HPLC, it is preferred to use a reverse phase on a strong anionic phase column.

The foregoing may be structurally depicted by the following schematic representation 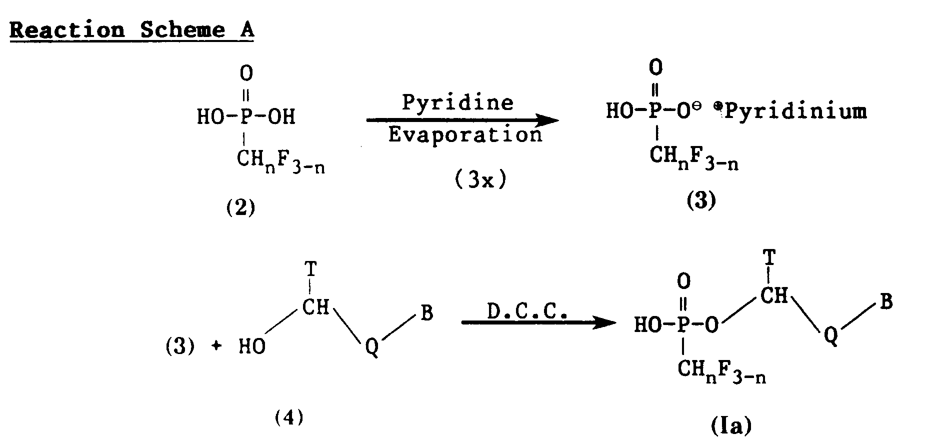 wherein n, Q, T and B are as defined in Formula Ia.

Of course, in those instances wherein there are reactive groups present on the T, Q and B moieties such groups may be protected with suitable protecting groups well known in the art. Following completion of the coupling reaction the protecting groups are readily removed. It is also obvious that, depending on the economics of any particular set of reactants, it may be just as convenient to separate any isomeric forms resulting from the coupling of the phosphonic acid derivatives (2) with an unprotected nucleoside of Formula (4); separation being effected by standard techniques (e.g. HPLC) well known in the art.

The preparation of the fluoromethyl phosphonic acid derivatives (2) is readily effected by procedures already known in the art. Preferably the appropriate mono-, di- or trifluoromethylphosphonic acids (2) are obtained by procedures outlined in J. Chem. Soc. Chem. Communication (1979) 739 (C.E. McKenna, J. Schmidhauser) and such as described in Synthesis 8, 615 (1979) and Tetrahedron Letters, 2323 (1983). These procedures are detailed in the specific exemplifications disclosed herein.

For the most part the nucleoside-bearing compounds of Formula 4, or their protected forms) are compounds which are already known in the prior art. In general these compounds are prepared by chemically coupling the appropriate carbohydrate with the appropriate base according to techniques well known and appreciated by those skilled in the art. A suitable teaching for the preparation of such nucleosides is the European Patent Application, publication No. 0184365 filed on November 25, 1985 and by the published U.S. Patent 4,146,715. In those instances wherein any specific base or carbohydrate is not previously described, such compounds may be prepared by processes and techniques analogously known in the art and by techniques illustrated in the herein disclosed examples for the preparation of novel acyclic moieties. Similarly the coupling of such bases and carbohydrates may be effected by standard procedures well known in the art. Of course, as is well taught in the above-cited references, in those instances wherein the preparation of novel nucleosides involves reactive moieties which may interfere with any reaction then such moieties must first be protected. This procedure, of course, is well understood by the ordinary chemist, elaborated herein-below.

The following examples I to XIII are illustrative of the preparation of compounds being similar to those of independent claim 1.

### Preparation of Intermediates

### Fluoromethyled Phosphonates and Fluoromethylated Phosphoric Acids

### Preparation A

### DIETHYL DIFLUOROMETHYLPHOSPHONATE

Under anhydrous conditions diethylphosphate (36 mmol), in 100 ml anhydrous tetrahydrofuran, is added dropwise to a suspension of sodium hydride (36 mmol) in 500 ml anhydrous tetrahydrofuran. After 0.5 h at room temperature, chlorodifluoromethane is allowed to bubble for 1 h into the reaction mixture cooled down to 0°C. After overnight stirring at 20°C, the reaction is quenched with water. Under work-up and distillation (50-60°C, 0.2 mmHg (26.6 Pa)) affords the expected compound (70% yield).

### Preparation B

### DIETHYL FLUOROMETHYLPHOSPHONATE

In a similar manner, by using chlorofluoromethane and following the teachings of the foregoing, there may also be produced diethyl fluoromethanephosphonate.

### Preparation C

### DIETHYL TRIFLUOROMETHYLPHOSPHONATE

Excess iodotrifluoromethane (2 ml) is condensed into a 5-ml flask containing triethyl phosphite (1.93 g, 12 mmol, freshly distilled from sodium metal). The reaction mixture is irradiated for 10 h with a Blak-Ray Long Wavelength Ultraviolet source (350.0 nm). After evaporation of the unreacted iodotrifluoromethane and the trifluoromethane by-product, distillation of the remaining residue gives diethyl trifluoromethanephosphonate (yield 1.2 g, 51%). b.p. 65-67.5°C.

### Preparation D

### DIFLUOROMETHYLPHOSPHONIC ACID

To diethyl difluoromethylphosphonate (10 g, 53 mmol) is added bromotrimethylsilane (133 mmol, 20.4 g). After overnight stirring and 1 h refluxing, the excess reagent is evaporated. Bistrimethylsilyl difluoromethylphosphonate is distilled (50-60°C, 0.2 mmHg) and to the distilled product is added 100 ml of water. The mixture is stirred and the solvent removed *in vacuo*. The expected compound is obtained as a white hydroscopic solid (90% yield). In a similar manner fluoromethylphosphonic acid and trifluoromethyl phosphoric acid are produced from their corresponding diethyl esters.

Preparation of Final Compounds of claim 1 is carried out in a similar manner as those illustrated in the following Examples.

### EXAMPLE I

### 9-[2,2,2-TRIFLUORO[1-(2-HYDROXYETHOXY)ETHYL]]guanine

### Ia) 2,2,2-TRIFLUORO-1-[2-(BENZYLOXY)ETHOXY]ETHANOL

Under anhydrous condition is added dropwise 1-ethoxy-2,2,2,-trifluoroethanol (28.84 g, 0.2 mol) to polyphosphoric acid (40 ml) at 150°-180°C. The so-formed gaseous 2,2,2-trifluoroacetaldehyde is condensated at -78°C. This product is then allowed to bubble into a solution of ethylenglycol monobenzyl ether (20 g, 0.132 mol) in anhydrous tetrahydrofuran (200 ml) at 0°C. After 1.5 h stirring at room temperature the solvent is evaporated and the compound obtained is used without purification in the next step.

### Ib) 2,2,2-TRIFLUORO-1-[2-(BENZYLOXY)ETHOXY]-ETHYL METHANE-SULFONATE

To 37.5 g of 2,2,2-trifluoro-1-[2-(benzyloxy)ethoxy]-ethanol in 200 ml of anhydrous dichloromethane, at 0°C, is added pyridine (16 ml, 0.22 mol) and then methanesulfonic anhydride (25.3 g, 0.145 mol). After stirring overnight at room temperature under a nitrogen atmosphere, the solvent is evaporated, the residue dissolved in diethyl ether, filtered, and the solvent evaporated. The compound is purified by flash chromatography on silica gel with 20% ethyl acetate/petroleum ether as eluant. Yield 33 g, 76%.

### Ic) 2-AMINO-6-CHLORO-9-[2,2,2-TRIFLUORO-1-[2-(BENZYLOXY)-ETHOXY]-ETHYL]PURINE

To 2-amino-6-chloropurine (3.1 g, 18.3 mmol) in suspension in anhydrous 1,2-dichloroethane (500 ml) is added bis(trimethylsilyl)acetamide (8.19 g, 40.3 mmol), and the mixture is refluxed for 1 h. To the refluxing mixture is added 2,2,2-trifluoro-1-[2-(benzyloxy)ethoxy]ethylmethanesulfonate (6 g, 18.3 mmol). After 17 h of reflux the mixture is poured into a saturated water solution of sodium bicarbonate and allowed to stand for 15 min. The mixture is filtered through celite, and the organic phase dried (MgSO₄) and evaporated.

By flash chromatography on silica gel, using a gradient of ethyl acetate/petroleum ether then ethyl acetate/ethanol as eluant, the N₇ and the N₉ isomer of the expected compound are separated.

After recrystallization in ethyl acetate/pentane 4% of the N₇ alkylated isomer and 0.9% of the N₉ alkylated isomer are obtained.

### Id) 9-[2,2,2-TRIFLUORO-1-[2-(BENZYLOXY)ETHOXY]ETHYL]GUANINE

2-amino-6-Chloro-9-[2,2,2-trifluoro-1-[2-(benzyloxy)ethoxy]-ethyl]guanine is heated for 15 h at 60°-70°C in a mixture of formic acid (30 ml), water (30 ml). The solvents are removed *in vacuo*. The residue is dissolved in water, treated with 0.15 ml of triethylamine and evaporated. The expected compound is obtained after recrystallization in methanol/diethyl ether/pentane. Yield 0.310 g, 82.5%.

### Ie) 9-[2,2,2-TRIFLUORO-1-(2-HYDROXYETHOXY)ETHYL]GUANINE

To 9-[2,2,2-trifluoro-1-[2-benzyloxyethoxy]-ethyl]guanine (0.287 g, 0.75 mmol) is added 10.5 ml cyclohexene, 20.5 ml ethanol and 0.07 g palladium hydroxide on carbon. The mixture is refluxed overnight then filtered. The solvents are removed *in vacuo*. The expected compound is obtained after recrystallization in ethyl alcohol/diethyl ether/pentane of the residue. Yield 0.19 g, 87%.

### EXAMPLE II

### 1-[2,2,2-TRIFLUORO-1-(2-HYDROXYETHOXY)ETHYL]CYTOSINE

### IIa) N⁴-ACETYL-1-[2,2,2-TRIFLUORO-1-[2-(BENZYLOXY)ETHOXY]ETHYL]CYTOSINE

Starting from 2,2,2-trifluoro-1-[2-(benzyloxy)ethoxy]ethyl methanesulfonate and N⁴-acetylcytosine and using the same procedure employed as in Step Ic) there is obtained the desired product. Yield 38%.

### IIb) 1-[2,2,2-TRIFLUORO-1-[2-(BENZYLOXY)ETHOXY]ETHYL]CYTOSINE

N⁴-Acetyl-1-[2,2,2-trifluoro-1-[2-(benzyloxy)ethoxy]ethyl]cytosine is dissolved in a methanol solution saturated with ammonia at 0°C. The mixture is stirred under pressure overnight at room temperature. After removal of the solvent *in vacuo*, the product is recrystallized from ethyl alcohol/diethyl ether.

### IIc) 1-[2,2,2-TRIFLUORO-1-(2-HYDROXYETHOXY)ETHYL]CYTOSINE

Starting from 1-[2,2,2-trifluoro-1-[2-(benzyloxy)ethoxy]ethyl]cytosine and using the same procedure employed as in Step Ie), there is obtained the desired product.

### EXAMPLE III

### 9-(2,2-DIFLUORO-4,5-DIHYDROXYPENTYL)GUANINE

### IIIa) 2,2-DIFLUORO-4-PENTENYL TRIFLUOROMETHANE SULFONATE

Under anhydrous conditions, at -10°C, to the solution of 2,2-difluoro-4-penten-1-ol (2.44 g, 20 mmol) in anhydrous dichloromethane (50 ml) is added pyridine (23 mmol, 1.86 ml), and then trifluoromethanesulfonic anhydride (1.82 g, 23 mmol). After 10 min at -10°C, the mixture is stirred 1 h at room temperature. The mixture is then poured into water (20 ml), the organic phase is washed with water (25ml) and brine (twice with 25 ml). After anhydrousing over magnesium sulfate and filtration the solvent is removed *in vacuo*. After distillation in the kugelrohr (12 mmHg) the expected compound is obtained. Yield 3.71 g, 83%.

### IIIb) 2-AMINO-6-CHLORO-9-(2,2-DIFLUORO-4-PENTENYL)PURINE

To a solution of 6-chloro-2-aminopurine (0.17 g, 1 mmol) in anhydrous dimethylformamide (10 ml) is added potassium carbonate (0.69 g, 5 mmol). After 10 min 2,2-difluoro-4-pentenyl-trifluoromethanesulfonate is added (0.224 g, 1 mmol). After 20 h at room temperature, the mixture is filtered and the solid washed with ethyl acetate. The solvents are removed *in vacuo*. By flash chromatography on silica gel with ethyl acetate as eluant, the expected compound is obtained as a white solid. Yield 0.197 g, 72%.

### IIIc) 2-AMINO-6-CHLORO-9-(2,2-DIFLUORO-4,5-DIHYDROXYPENTYL)PURINE

To 2-Amino-6-chloro-9-(2,2-difluoro-4-pentenyl)purine (0.135 g, 0.5 mmol) in solution, in a mixture of acetone (5 ml) and water (0.15 ml) is added 4-methylmorpholine-4-oxyde (0.078 g, 0.665 mmol), and osmium tetroxide (0.08 ml of a 0.1% solution in t-butanol). After 15 h reflux the mixture is poured in water. Sodium thiosulfate is added to destroy the remaining osmium tetroxide. After 10 min of stirring, the solvents are evaporated. The residue is purified by flash chromatography on silica gel with 20% ethyl alcohol/ethyl acetate as eluant. The expected compound is obtained by recrystallization from ethyl alcohol. Yield 66%.

### IIId) 9-(2,2-DIFLUORO-4-HYDROXYBUTYL)GUANINE

2-Amino-6-chloro-9-(2,2-difluoro-4,5-dihydroxypentyl)purine (0.784 g, 2.55 mmol) is refluxed with 50 ml of hydrochloric acid (2M) for 3 h. After evaporation of the solvent and recrystallization the title compound is obtained as a hydrochloric salt. Yield 96%.

### EXAMPLE IV

### 1-(2,2-DIFLUORO-4-HYDROXYBUTYL)CYTOSINE

### IVa) 2,2-DIFLUORO-4-PENTEN-1-OL

Under anhydrous conditions, to a cooled solution (10-15°C) of sodium borohydride (5.08 g, 135 mmol) in 100 ml of ethyl alcohol is added dropwise ethyl-2,2-difluoro-4-pentenoate (28.64 g, 175 mmol). After 2 h stirring at room temperature, the reaction mixture is quenched at 0°C with 100 ml of 1M sulfuric acid. Dichloromethane (300 ml) is added, and the phases separated. The aqueous phase is extracted with more dichloromethane and the organic phases are washed with brine. By fractional distillation the expected compound is separated from the solvents. (bp 55-60°C/15 mmHg - yield 70%).

### IVb) 2-(2,2-DIFLUORO-4-PENTENYLOXY)TETRADYDROPYRAN

At 0°C under anhydrous conditions, to a solution of 2,2-difluoro-4-penten-1-ol (13 g, 0.107 mmol) and a catalytic amount of p-toluenesulfonic acid in anhydrous dichloromethane (250 ml) is added dihydropyran (13.43 g, 0.16 mmol). The reaction is stirred overnight at room temperature, then washed with a sodium bicarbonate solution and brine. The organic phase is dried over magnesium sulfate and the solvent removed *in vacuo*.

The expected compound is obtained after flash chromatography on silica gel with 1% ethyl acetate/petroleum ether as eluant. Yield 19.43 g, 94%.

### IVc) 3,3-DIFLUORO-4-TETRAHYDROPYRANYLOXY-1-BUTANOL

At -78°C ozone is bubbled into a solution of 2-(2,2-difluoro-4-pentenyloxy)tetrahydropyran (10 g, 51.5 mmol) in anhydrous dichloromethane (120 ml) and ethyl alcohol (120 ml), until the blue color persists. After quenching the reaction with 2 ml methyl sulfide, the mixture is transferred by nitrogen pressure into a solution of sodium borohydride (2.16 g, 0.057 mol) in ethyl alcohol (100 ml) at -20°C to -10°C.

After stirring for 4 h at room temperature the reaction is quenched with acetone, and the solvents evaporated. The residue is dissolved in dichloromethane and washed with a solution of ammonium chloride in water, the organic phase is dried over magnesium sulfate, and the solvent is removed *in vacuo*. The compound so obtained is used without any further purification. Yield 7.27 g, 71%.

### IVd) 2-(4-BENZYLOXY-2,2-DIFLUOROBUTOXY)TETRAHYDROPYRAN

Under anhydrous conditions, to 3,3-difluoro-4-tetrahydropyranyloxy-1-butanol (7.07 g, 34 mmol) and benzyl bromide (6.72 g, 39 mmol) in solution in anhydrous tetrahydrofuran (120 ml) is added in several times potassium tert-butoxide (4.41 g, 39 mmol). The reaction mixture is stirred overnight at room temperature, then quenched with an aqueous solution of ammonium chloride. After the usual work-up and purification by flash chromatography on silica gel with 10% ethyl acetate/petroleum ether as eluant the expected compound is obtained.

### IVe) 4-BENZYLOXY-2,2-DIFLUORO-1-BUTANOL

To 2-(4-benXyloxy-2,2-difluorobutoxy)tetrahydropyrane(10.263 g, 34 mmol) dissolved in methyl alcohol is added a catalytic amount of p-toluenesulfonic acid. After overnight stirring at room temperature the solvent is removed *in vacuo*. After purification by flash chromatography on silica gel using 10% diethyl ether/petroleum ether as eluant the expected compound is obtained. Yield 5.18 g, 82%.

### IVf) 4-BENZYLOXY-2,2-DIFLUOROBUTYLTRIFLUOROMETHANE SULFONATE

Under anhydrous conditions, at 0°C, to a 4-benzyloxy-2,2-difluoro-1-butanol (5 g, 23 mmol) in solution in anhydrous dichloromethane (160 ml) and pyridine (2.75 g, 35 mmol), is added dropwise a solution of trifluoromethanesulfonic anhydride in dichloromethane (50 ml). The reaction is stirred overnight at room temperature, then washed with water. The organic phase is dried and the solvent removed *in vacuo*. The expected compound obtained (7.86 g, 97%) is used without any further purification.

### IVg) N⁴-ACETYL-1-[4-BENZYLOXY-2,2-DIFLUOROBUTYL]CYTOSINE

Starting from IVf) and N⁴-acetylcytosine and using the same procedure employed as in step IIIb) there is obtained the desired product.

### IVh) 1-[4-BENZYLOXY-2,2-DIFLUOROBUTYL]CYTOSINE

Starting from IVg) and using the same procedure employed as in step IIb) there is obtained the desired product.

### IVi) 1-(2,2-DIFLUORO-4-HYDROXYBUTYL)CYTOSINE

Starting from IVh) and using the same procedure employed as in stepI there is obtained the desired product.

### EXAMPLE V

### 1-[2,2-DIFLUORO-1-(2-HYDROXYETHOXY)-3-HYDROXYPROPYL]CYTOSINE

### Va) ETHYL 2,2-DIFLUORO-3-HYDROXYPROPANOATE

Under anhydrous conditions the mixture of paraformaldehyde (0.3 g, 3.3 mmol), activated zinc powder (0.715 g, 11 mmol) and 15 ml of tetrahydrofuran is refluxed. The ethyl bromodifluoroacetate (2.03 g, 10 mmol) dissolved in 15 ml tetrahydrofuran is added dropwise. After 30 min reflux the reaction is quenched with an aqueous solution of ammonium chloride. The usual work-up followed by flash chromatography on silica gel with 35% diethyl ether/petroleum ether as eluant affords the expected compound. Yield 200 mg, 13%.

### Vb) ETHYL 2,2-DIFLUORO-3-BENZYLOXYPROPANOATE

Under anhydrous conditions, to the mixture of ethyl 2,2-difluoro-3-hydroxypropanoate (0.135 g, 0.88 mmol), benzylbromide, tetrabutylammonium iodide (0.324 g, 0.88 mmol) and dimethylformamide (3 ml), is added at -10°C sodium hydride (0.044 g, 0.92 mmol). After 40 h stirring at room temperature the reaction is quenched with water. After the usual work-up, and flash chromatography on silica gel with 15% ethyl acetate/petroleum ether as eluant, the expected compound is obtained. Yield 0.114 g, 53%.

### Vc) 2,2-DIFLUORO-3-BENZYLOXY PROPANAL

Under anhydrous conditions, to ethyl 2,2-difluoro-3-benzyloxypropanoate (1.14 g, 4.7 mmol) in 10 ml diethyl ether is added (0.45 g, 11.75 mmol) of lithium aluminium hydride. After stirring 1 h at room temperature are added 45 mg water, then 45 mg of a 15% aqueous solution of sodium hydroxyde, then 135 mg of water. After drying over magnesium sulfate, filtration and evaporation of the solvent the product is distilled just before further utilization.

### Vd) 3-BENZYLOXY-1-(2-BENZYLOXYETHOXY)-2,2-DIFLUORO-1-PROPANOL

An equimolar mixture of 2,2-difluoro-3-benzyloxy propanal and ethylenglycolmonobenzyl ether are dissolved in anhydrous tetrahydrofuran. Reaction time: 15 min at room temperature. After evaporation of the solvent and flash chromatography on silica gel (eluant: 20% ethyl acetate/petroleum ether) the expected compound is obtained.

### Ve) 3-BENZYLOXY-1-(2-BENZYLOXYETHOXY)-2,2-DIFLUOROPROPYL METHANESULFONATE

Under anhydrous conditions, at 0°C, to 3-benzyloxy-1-(2-benzyloxyethoxy)-2,2-difluoro-1-propanol (0.31 g, 0.88 mmol), pyridine (0.25 ml) and anhydrous dichloromethane (3 ml) is added dropwise a solution of methanesulfonic anhydride (0.153 g, 0.88 mmol) in 2 ml dichloromethane. After stirring 20 h at room temperature, the reaction is quenched with water, the organic phase washed with 0.1M hydrochloric acid, dried and the solvent is removed *in vacuo*. Purification by flash chromatography on silica gel using 20% ethyl acetate/petroleum ether as eluant gives the expected compound.

### Vf) N⁴-ACETYL-1-[3-BENZYLOXY-1-(2-BENZYLOXYETHOXY)-2,2-DIFLUORO-1-PROPYL]CYTOSINE

Starting from Ve) and N⁴-acetylcytosine and using the same procedure employed as in Step Ic) there is obtained the desired product.

### Vg) 1-[3-BENZYLOXY-1-(2-BENZYLOXYETHOXY)-2,2-DIFLUORO-1-PROPYL]CYTOSINE

Starting from Vf) and using the same procedure employed as in Step IIb) there is obtained the desired product.

### Vh) 1[2,2-DIFLUORO-1-(2-HYDROXYETHOXY)-3-HYDROXYPROPYL]CYTOSINE

Starting from Vg) and using the same procedure employed as in Step I there is obtained the desired product.

### EXAMPLE VI

### 9-(2,2-DIFLUORO-4-HYDROXYBUTYL)GUANINE

### VIa) 2-AMINO-6-CHLORO-9-(4-BENZYLOXY-2,2-DIFLUORO-4-HYDROXYBUTYL)PURINE

Starting from IVe) and 2-amino-6-chloroguanine, and using the same procedure employed as in Step IIIb) there is obtained the desired product.

### VIb) 9-(2,2-DIFLUORO-4-HYDROXYBUTYL)GUANINE

2-Amino-6-chloro-9-(4-benzyloxy-2,2-difluoro-4-hydroxybutyl)purine (0.193 g, 0.52 mmol) is refluxed in 2M hydrochloric acid for 10 h. The mixture is evaporated to dryness, and the residue recrystallized in water/ethyl alcohol after treatment with propylen oxyde. The expected compound is obtained in a good yield: 0.073 g, 64%.

### EXAMPLE VII

### 9-[2,2-DIFLUORO[1-(2-HYDROXYETHOXY)ETHYL]]GUANINE

### VIIa) 2,2-DIFLUORO-1-[2-(BENZYLOXY)ETHOXY]ETHANOL

Starting from 2,2-difluoro-1,1-ethanediol and using the same procedure employed as in step Ia) there is obtained the desired product.

### VIIb) 2,2-DIFLUORO-1-[2-(BENZYLOXY)ETHOXY]ETHYLMETHANESULFONATE

Starting from VIIa) and using the same procedure employed as in step Ib) there is obtained the desired product.

### VIIc) 2-AMINO-6-CHLORO-9-[2,2-DIFLUORO-1-[2-(BENZYLOXY)ETHOXY]ETHYL]PURINE

Starting from VIIb) and using the same procedure employed as in step Ic) there is obtained the desired product.

### VIId) 9-[2,2-DIFLUORO-1-[2-(BENZYLOXY)ETHOXY]ETHYL]GUANINE

Starting from VIIc) and using the same procedure employed as in step Id) there is obtained the desired product.

### VIIe) 9-[2,2-DIFLUORO[1-(2-HYDROXYETHOXY)ETHYL]]GUANINE

Starting from VIId) and using the same procedure employed as in step Ie) there is obtained the desired product.

### EXAMPLE VIII

### 1-[2,2-DIFLUORO-1-(2-HYDROXYETHOXY)ETHYL]CYTOSINE

### VIIIa) N⁴-ACETYL-1-[2,2-DIFLUORO-1-[2-(BENZYLOXY)ETHOXY]ETHYL]CYTOSINE

Starting from VIIb) and N⁴-acetyl cytosine and using the same procedure employed as in step Ic) there is obtained the desired product.

### VIIIb) 1-[2,2-DlFLUORO-1-[2-(BENZYLOXY)ETHOXY]ETHYL]CYTOSINE

Starting from VIIIa) and using the same procedure employed as in step IIb) there is obtained the desired product.

### VIIIc) 1-[2,2-DIFLUORO-1-(2-HYDROXYETHOXY)ETHYL]CYTOSINE

Starting from VIIIb) and using the same procedure employed as in step Ie) there is obtained the desired product.

### EXAMPLE IX

### 1-[(1,1'-DIFLUORO-2,4-DIHYDROXY-3-BUTOXY)METHYL]CYTOSIN

### IXa) 3,4-O-ISOPROPYLIDENE-2-BENZYLOXY-1-(1,3-DITHIANE)-3,4-BUTANEDIOL

A solution of 3,4-O-isopropylidene-1-(1,3-dithiane)-2,3,4-butanetriol (2.5 g, 10 mmol) in anhydrous tetrahydrofuran (20 ml) is added to a suspension of sodium hydride (0.55 g, 11 mmol, 50% in oil) under argon. The mixture is stirred at room temperature until no gas evolution is detected (about 30 min). Then a solution of benzyl chloride (1.2 g, 10 mmol) and tetra-n-butylammonium iodide (0.06 g) in tetrahydrofuran (10 ml) is added. The mixture is stirred 12 h at room temperature and then hydrolyzed with an aqueous solution of ammonium chloride. After usual work-up the product is purified by flash chromatography on silica gel. (ethyl acetate:hexane, 2:8) (1.5 g).

### IXb) 3,4-O-ISOPROPYLIDENE-2-BENZYLOXY-3,4-DIHYDROXYBUTYRALDEHYDE

A solution of 3,4-O-isopropylidene-2-benzyloxy-1-(1,3-dithiane)-3,4-butanediol (8 g, 23.5 mmol) in tetrahydrofuran (20 ml) is added dropwise during 10 min to a well stirred suspension of mercury^{II} oxide (10.5 g, 45 mmol) and boron trifluoride etherate (6 ml, 47 mmol) in tetrahydrofuran:H₂O (8:2). The mixture is stirred 24 h at room temperature, then neutralized with an aqueous solution of sodium bicarbonate, diluted with diethyl ether and filtered off through a path of celite. After usual work-up the product is purified by flash chromatography on silica gel (ethyl acetate:hexane, 2:8) (3.3 g).

### IXc) 1,1'-DIFLUORO-3,4-O-ISOPROPYLIDENE-2-BENZYLOXY-3,4-BUTANEDIOL

A sample of dimethylaminosulfur trifluoride (1.6 ml, 13 mmol) is added to a solution of 3,4-O-isopropylidene-2-benzyloxy-3,4-dihydroxy-butyraldehyde (3.3 g, 13 mmol) in anhydrous dichloromethane (100 ml) under nitrogen. The mixture is stirred 24 h at room temperature, neutralized with 10% aqueous sodium bicarbonate and extracted with dichloromethane. After usual work-up the product is purified by flash chromatography on silica gel (ethyl acetate:hexane, 8:2) (3.3 g).

### IXd) 1,1'-DIFLUORO-2-BENZYLOXY-3,4-BUTANEDIOL

1,1'-difluoro-3,4-O-isopropylidene-2-benzyloxy-3,4- butanediol (2.7 g, 10 mmol) is dissolved in a mixture of acetic acid:H₂0; 8:1 (10 ml) and heated at 50°C for 1.5 h. The reaction mixture is then concentrated and the product purified by flash chromatography on silica gel (ethyl acetate:hexane, 1:1) (2.2 g).

### IXe) 1,1'-DIFLUORO-2-BENZYLOXY-4-t-BUTYLDIPHENYLSILYLOXY-3-BUTANOL

A solution of t-butyldiphenylchlorosilane (2.7 g, 1 mmol) in dichloromethane (5 ml) is added to a solution of 1,1'-difluoro-2-benzyloxy-3,4-butanediol (2.3 g, 1 mmol), 4-dimethylaminopyridine (0.05 g) and triethylamine (0.65 ml, 1 mmol) in dichloromethane (25 ml). The mixture is stirred 12 h at room temperature and then, after usual work-up, the product is purified by flash chromatography on silica gel (ethyl acetate:hexane, 85:15) (3.8 g).

### IXf) 1,1'-DIFLUORO-2-BENZYLOXY-4-t-BUTYLDIPHENYLSILYLOXY-3-TETRAHYDROPYRANYLOXY BUTANE

A mixture of 1,1'-difluoro-2-benzyloxy-4-t-butyldiphenylsilyloxy-3-butanol (2.35 g, 5 mmol), dihydropyran (5 ml), pyridinium paratoluenesulfonate (0.1 g) in anhydrous dichloromethane (15 ml) is stirred 12 h at room temperature under nitrogen. After usual work-up the product is purified by flash chromatography on silica gel (ethyl acetate:hexane, 1:9) (1.7 g).

### IXg) 1,1'-DIFLUORO-2-BENZYLOXY-3-TETRAHYDROPYRANYLOXY-4-BUTANOL

A 1M solution of tetra-n-butyl ammonium fluoride in tetrahydrofuran (13 ml, 13 mmol) is added to a solution of 1,1'-difluoro-2-benzyloxy-4-t-butyldiphenylsilyloxy-3-tetra-hydropyranyloxy butane (3.5 g, 63 mmol) in tetrahydrofuran (30 ml) at room temperature under nitrogen. The mixture is stirred overnight and after usual work-up the product is purified by flash chromatography on silica gel (ethyl acetate:hexane, 25:75) (2 g).

### IXh) 1,1'-DIFLUORO-2,4-DIBENZYLOXY-3-TETRAHYDROPYRANYLOXY BUTANE

A solution of 1,1'-difluoro-2-benzyloxy-3-tetrahydro-pyranyloxy-4-butanol in anhydrous tetrahydrofuran (15 ml) is added to a suspension of sodium hydride (0.35 g, 50% in oil, 63 mmol) in anhydrous tetrahydrofuran (20 ml) at room temperature under nitrogen. After completion evolution of gas (about 30 min) a solution of benzylbromide (1.1 g, 63 mmol) is added. The mixture is stirred 48 h at room temperature and then, after usual work-up, the product is purified by flash chromatography on silica gel (ethyl acetate:hexane, 15:85) (2.2 g).

### IXi) 1,1-DIFLUORO-2,4-DIBENZYLOXY-3-BUTANOL

A solution of 1,1'-difluoro-2,4-dibenzyloxy-3-tetrahydropyranyloxy butane (2.2 g, 5.5 mmol) in absolute ethyl alcohol (70 ml) and p-toluenesulfonic acid (0.2 g) is left overnight at room temperature under nitrogen. After evaporation of the solvent and usual work-up the product is purified by flash chromatography on silica gel (ethyl acetate:hexane, 8:2) (1.8 g).

### IXj) 1-[1,1'-DIFLUORO-2,4-DIBENZYLOXY-3-BUTOXY)METHYL]-N⁴-ACETYLCYTOSINE

A stream of anhydrous HCl gas is bubble rapidly in a solution of 1,1'-difluoro-2,4-dibenzyloxy-3-butanol (1.1 g, 5.4 mmol) and paraformaldehyde (0.2 g, 0.07 mol) in anhydrous 1,2-dichloroethane (30 ml) at 0°C during 5 min and then at a slower rate during 4 h. The mixture is then left overnight at 0°C, purged with nitrogen, dried over MgSO₄, filtered and concentrated *in vacuo*. The residue is dissolved in anhydrous 1,2-dichloroethane (5 ml) and added to a solution of bistrimethylsilyl N4-acetyl-cytosine (1.05 g, 3.4 mmol) and tetra-n-butyl ammonium fluoride (0.1 g, 0.3 mmol) in anhydrous 1,2-dichloroethane (10 ml). The mixture is heated 5 h under argon and then hydrolyzed by sequential addition of methyl alcohol (2 ml) and H₂0 (2 ml) for a few minutes and then extracted with dichloromethane. The product is purified by flash chromatography on silica gel (methyl alcohol:dichloromethane, 2:98) (0.45 g), m.p. 110°C.

### IXk) 1-[(1,1'-DIFLUORO-2,4-DIBENZYLOXY-3-BUTOXY)METHYL]CYTOSINE

A solution of 1-[(1,1'-difluoro-2,4-dibenzyloxy-3-butoxy)methyl]N⁴-acetylcytosine (0.12 g, 0.0025 mol) in absolute ethyl alcohol (10 ml) is saturated with anhydrous NH₃ at 0°C. The flask is then sealed and left 12 h at room temperature. The mixture is then cooled to allow the opening of the scelled flask and the solvent is evaporated *in vacuo*. The product is purified by flash chromatography on silica gel (methyl alcohol:dichloromethane, 5:95) (0.06 g).

### IXl) 1-[(1,1'-DIFLUORO-2,4-DIHYDROXY-3-BUTOXY)METHYL]CYTOSINE

A 1M solution of BCl₃ (boron trichloride) (0.15 ml, 1.5 mmol) in dichloromethane is added to a solution of 1-[(1,1'-difluoro-2,4-dibenzyloxy-3-butoxy)methyl]cytosine (0.06 g, 0.135 mmol) in anhydrous dichloromethane at -78°C under nitrogen. The solution mixture is stirred at 2 h at -78°C and a 1M solution of boron trichloride (0.15 ml) is added. The mixture is stirred one additional hour and then a mixture of methyl alcohol:dichloromethane, 1:1 (1.5 ml) is added and the solution is allowed to warm at room temperature. The mixture is then concentrated *in vacuo* to afford the title compound (0.03 g).

### EXAMPLE X

### 2-[(9-GUANYL)METHOXY]ETHYL DIFLUOROMETHYL PHOSPHONATE

9-[(2-hydroxyethoxy)methyl]guanine (0.1 g, 0.444 mmol) and difluoromethyl phosphonic acid (0.131 g, 0.89 mmol) are dissolved in 2 ml of anhydrous pyridine and the solvent is removed *in vacuo* at 35°C. This procedure is repeated 3 times to provide an anhydrous reaction mixture. The residue is dissolved in 5 ml of anhydrous pyridine and dicyclohexyl-carbodiimide (0.367 g, 1.78 mmol) is added. After stirring at 35°C under a nitrogen atmosphere for 20 h, 10 ml of water are added, and the mixture is stirred for 1/2 h. The mixture is filtered, the solid washed with water and the solvent is evaporated. Water (5 ml) is added and removed *in vacuo*. This procedure is repeated to eliminate the remaining pyridine. The residue is dissolved in 5 ml acetic acid (10% in water) and heated at 100°C for 1.5 h. The solvent is evaporated. The yield after recrystallization of the residue in water/ethyl alcohol is 0.084 g, 56%.

This product is converted into its ammonia salt by treating it with concentrated ammonia and recrystallized in water/ethyl alcohol.

### EXAMPLE XI

### 1,1-DIFLUORO-4-HYDROXYBUTYL-1,4-PHOSPHONOLACTONE

### XIa) DIETHYL-1,1-DIFLUORO-4-TETRAHYDROPYRANYLOXY PHOSPHONATE

At -78°C, to a solution of lithium diisopropylamide (3.3 mmol) in 30 ml of tetrahydrofuran is added diethyl difluoromethylphosphonate (3 mmol, 0.564 g) diluted with 2 ml of tetrahydrofuran. After 15 min is added 1-iodo-3-tetrahydropyranyloxypropane (3 mmol, 0.810 g) in 2 ml of tetrahydrofuran. The reaction mixture is quenched with an aqueous solution of ammonium chloride after 2 hours stirring. After usual work-up, followed by flash chromatography purification on silica gel with 10% ethyl acetate/ petroleum ether as eluant, the expected compound is obtained. Yield 60%.

### XIb) DIETHYL-1,1-DIFLUORO-4-HYDROXYBUTYL PHOSPHONATE

A catalytic amount of paratoluene sulfonic acid is added to a solution of diethyl-1,1-difluoro-4-tetrahydropyranyloxy phosphonate (1.8 mmol, 0.588 g) in methyl alcohol (40 ml). After overnight stirring at room temperature and usual work-up followed by flash chromatography purification on silica gel with 70% ethyl acetate/petroleum ether as eluant, the expected compound is obtained. Yield 87%.

### XIc) 1,1-DIFLUORO-4-HYDROXYBUTYL PHOSPHONIC ACID

Bromotrimethylsilane (0.717 g, 4.7 mmol) is added to diethyl-1,1-difluoro-4-hydroxybutyl phosphonate (0.384 g, 1.56 mmol). After 48 hours of stirring the volatile part is removed by evaporation *in vacuo* and the residue is stirred overnight with 10 ml water. Filtration followed by evaporation *in vacuo* affords the expected compound. Yield 95%.

### XId) 1,1-DIFLUORO-4-HYDROXYBUTYL-1,4-PHOSPHONOLACTONE

1,1-Difluoro-4-hydroxybutyl phosphonic acid (0.235 g, 1.24 mmol) is dissolved in 2 ml of anhydrous pyridine and the solvent is removed *in vacuo* at 35°C. This procedure is repeated 3 times to provide an anhydrous reaction mixture. The residue is dissolved in 10 ml of anhydrous pyridine, and dicyclohexylcarbodiimide (1.24 mmol, 0.256 g) is added. After stirring 24 hours at room temperature under a nitrogen atmosphere, 30 ml of water is added, and the mixture is stirred for ½ hour. The mixture is filtered, the solid washed with water and the solvents are evaporated. The residue is purified by ion exchange chromatography on DOWEX AG® 1X4 (HCOO⁻ form). The expected compound is obtained. Yield 70%.

### EXAMPLE XII

### 1,1-DIFLUORO-3-HYDROXYPROPYL-1,3-PHOSPHONOLACTONE

### XIIa) DIETHYL-1,1-DIFLUORO-3-TETRAHYDROPYRANYLOXYPROPYL PHOSPHONATE

Starting from diethyl difluoromethanephosphonate and 1-iodo-2-tetrahydropyranyloxy propane and using the same procedure as in Step XIa), the desired product is obtained.

### XIIb) DIETHYL-1,1-DIFLUORO-3-HYDROXYBUTYL PHOSPHONATE

Starting from XIIa) and using the same procedure as in Step XIb), the desired product is obtained.

### XIIc) 1,1-DIFLUORO-3-HYDROXYBUTYL PHOSPHONATE

Starting from XIIb) and using the same procedure as in step XIc) the desired product is obtained.

### XIId) 1,1-DIFLUORO-3-HYDROXYBUTYL-1,3-PHOSPHONOLACTONE

Starting from XIIc) and using the same procedure as in step XId) the desired product is obtained.

### EXAMPLE XIII

### 1,1-DIFLUORO-3-(9-GUANIDYL)METHOXY-4-HYDROXYBUTYL-1,4-PHOSPHONOLACTONE

### XIIIa) DIETHYL-1,1-DIFLUORO-3-BUTENYL PHOSPHONATE

Starting from diethyl difluoromethanephosphonate and allyl bromide and using the same procedure as in step XIIa) the desired product is obtained.

### XIIIb) DIETHYL-1,1-DIFLUORO-3,4-EPOXYBUTYL PHOSPHONATE

At 0°C to a solution of XIIIa) (0.228 g, 1 mmol) in 10 ml dichloromethane is added metachloroperbenzoic acid (0.172 g, 1 mmol). After overnight stirring at room temperature and usual work-up, followed by flash chromatography purification, the desired compound is obtained. Yield 80%.

### XIIIc) DIETHYL-1,1-DIFLUORO-3-HYDROXY-4-BENZYLOXYBUTYL PHOSPHONATE

To a suspension of sodium hydride (0.048 g, 2 mmol) in tetrahydrofuran (10 ml) is added anhydrous benzyl alcohol (0.216 g, 2 mmol) in 1 ml tetrahydrofuran. After 1 hour stirring at room temperature XIIIb) is added (0.244 g, 2 mmol). The reaction mixture is refluxed for 5 hours, then quenched with brine. After the usual work-up, followed by flash chromatography purification, the desired compound is obtained. Yield 60%.

### XIIId) DIETHYL-1,1-DIFLUORO-3-CHLOROMETHOXY-4-BENZYLOXYBUTYL PHOSPHONATE

To a solution of XIIIc) (3.49 g, 10 mmol) in anhydrous dichloromethane (10 ml) is added paraformaldehyde (0.45 g). In this mixture maintained at 0°C is bubbled gazeous hydrochloric acid until the paraformaldehyde is completely dissolved. After 48 hours at 0°C the reaction mixture is dried with calcium chloride, filtrated and the solvent is evaporated *in vacuo*. The expected product is obtained in 83% yield and used without further purification.

### XIIIe) DIETHYL-1,1-DlFLUORO-3-[9(2-AMINO-6-CHLORO)PURINYL]-METHOXY-4-BENZYLOXYBUTYL PHOSPHONATE

To a solution of XIIId) (0.397 g, 1 mmol) in anhydrous dimethylformamide is added triethylamine (2 mmol, 0.102 g) and 2-amino-6-chloropurine (0.170 g, 2 mmol). After overnight stirring at room temperature the solvent is removed *in vacuo*. Usual work-up and purification by flash chromatography affords the expected compound. Yield 22%.

### XIIIf) DIETHYL-1,1-DIFLUORO-3-(9-GUANIDYL)METHOXY-4-BENZYLOXYBUTYL PHOSPHONATE

A solution of XIIIe) (0.531 g, 1 mmol) in 10 ml of a 50% solution of formic acid in water, is heated overnight at 60°C. After evaporation *in vacuo* and purification by flash chromatography the desired compound is obtained. Yield 85%.

### XIIIg) DIETHYL-1,1-DIFLUORO-3-(9-GUANIDYL)METHOXY-4-HYDROXYBUTYL PHOSPHONATE

Starting from XIIIf) and using the same procedure as in step Ib the desired compound is obtained. Yield 85%.

### XIIIh) 1,1-DIFLUORO-3-(9-GUANIDYL)METHOXY-4-HYDRDXYBUTYL PHOSPHONATE

Starting from XIIIg) and using the same procedure as in step XIc) the desired compound is obtained. Yield 91%.

### XIIIi) 1,1-DiFLUORO-3-(9-GUANIDYL)METHOXY-4-HYDROXYBUTYL-1,4-PHOSPHONOLACTONE

Starting from XIIIh) and using the same procedure as in step XId) the desired compound is obtained. Yield 82%.

Similarly, by replacing the 9-[(2-hydroxy-ethoxy)methyl]guanine, used as a reactant in Example X, with equivalent quantities of the alcohols prepared in Examples I to IX, and by substantially following the procedure of Example X, there will be produced the corresponding difluoromethyl phosphonate derivatives, i.e.
1) 2-[1-(9-guanyl)-2,2,2-trifluoroethoxy]ethyl difluoromethylphosphonate
2) 2-[1-(1-cytosyl)-2,2,2-trifluoroethoxy]ethyl difluoromethylphosphonate
3) 5-(9-guanyl)-4,4-difluoro-2-hydroxypentyl difluoromethylphosphonate
4) 4-(1-cytosyl)-3,3-difluorobutyl difluoromethylphosphonate
5) 2-[1-(1-cytosyl)-2,2-difluoro-3-hydroxypropoxy)ethyl difluoromethylphosphonate
6) 4-(9-guanyl)-3,3-difluorobutyl difluoromethylphosphonate
7) 2-[1-(9-guanyl)-2,2-difluoroethoxy]ethyl difluoromethylphosphonate
8) 2-[1-(1-cytosyl)-2,2-difluoroethoxy]ethyl difluoromethylphosphonate
9) 2-(1-cytosyl)methoxy]-3,-hydroxy-4,4-difluorobutyl difluoromethylphosphonate...

Similarly, by following the procedure of example XIII and in using the appropriate starting materials the compounds as claimed in claim 1 are obtained.

It will be understood that the reaction in which the fluoromethylphosphonic acid of this invention is coupled in 5' with the nucleoside or analog, is frequently of a nature such that the other hydroxy groups must be protected to keep them from reacting with the fluoromethylphosphonic acid, or being decomposed in some manner. The protecting groups are chosen from the groups used in synthetic organic chemistry for the purpose. Chemists are accustomed to choosing groups which can be efficiently placed on hydroxy groups, and which can be easily removed when the desired reaction is complete. Suitable groups are described in standard textbooks, such as Chapter 3 of Protective Groups in Organic Chemistry, McOmie, Ed., Plenum Press, New York (1973); and Chapter 2 of Protective Groups in Organic Synthesis, Greene, John Wiley and Sons, New York (1981).

Typical hydroxy-protecting groups include formyl, 2-chloroacetyl, benzyl, diphenylmethyl, triphenylmethyl, 4-nitrobenzyl, phenoxycarbonyl, t-butyl, methoxymethyl, tetrahydropyranyl, allyl, tetrahydrothienyl, 2-methoxyethoxymethyl, methoxyacetyl, phenoxyacetyl, isobutyryl, ethoxycarbonyl, benzyloxycarbonyl and the like. Silyl hydroxy-protecting groups are often particularly convenient, because most of them are easily selectively cleaved with fluorine anion. Such groups include especially trimethylsilyl, as well as isopropyldimethylsilyl, methyldiisopropylsilyl, triisopropylsilyl and the like. The t-butyldimethylsilyl and t-butyldiphenylsilyl groups are special cases and are preferred as the protecting groups in this synthesis.

By following the esterification techniques described in the foregoing examples for the coupling of the fluorinated methyl phosphonic acid derivatives to the nucleosides, (as well as the application of the techniques generally available to those of ordinary skill in the art analogously known for achieving this purpose) and by substitution of the above nucleosides with equivalent quantities of the appropriate prior art nucleosides there will be produced the 5'-fluoromethylphosphonate, the 5'-difluoromethylphosphonate and the 5'-trifluoromethylphosphonate of each of the following specific compounds of Formula Ia:
Uridine
3'-Azido-3'-deoxythymidine
3'-Deoxythymidine
3'-Deoxythymidine-2'-ene
3'-Azido-2'3'-dideoxy-5-bromouridine
3'-Azido-2'3'-dideoxy-5-fluorouridine
3'-Azido-2'3'-dideoxy-5-chlorouridine
3'-Azido-2'3'-dideoxy-5-iodouridine
2'-Deoxy-5-fluorouridine
2'-Deoxy-5-bromouridine
2'-Deoxy-5-iodouridine
2'-Deoxyuridine
(E)-5-(2-bromovinyl)-2'-deoxyuridine
5-Trifluoromethyl-2'-deoxyuridine
5-(2-Chloroethyl)-2'-deoxyuridine

The following compounds also may be made by said coupling reaction:
2-[1-(1-(5-Fluorouracil))methoxyethyl]mono-, di- and trifluoromethylphosphonates

The compounds of this invention (Formula I) are useful as antiviral and antitumoral agents.

More specifically the compounds of Formula I possess the inherent property of having antiviral activity against various classes of DNA and RNA viruses *in vitro* and *in vivo* experimental models. Particular viruses are cytomegalovirus,adenovirus, particularly adenovirus 5, Mengo virus and Sindbar virus. Significant activity is to be found against vaccinia, and herpes viruses such as simplex, yoster, and varicella, in mammals, which cause diseases as for example herpetic keratitis in rabbits and herpetic encephalitis in mice and bovine rhinotrachiitis virus. Included within the scope of the term "anti-viral" are retroviruses such as human immuno-deficient viruses (e.g. HIV), the specific retroviruses being well known in the art. Standard antiviral screens and techniques may be used to assess the antiviral activity of the compounds of Formula I and the results compared to agents known to possess antiviral activity. To achieve their anti-viral effects, the compounds of this invention may be administered (calculated as the free base) at about 0.1 to 250 mg/kg, preferably 1.0 to 50 mg/kg of mammal body weight, administered one to four times daily.

In addition to their use as antiviral agents, the compounds of this invention provide a method for treating susceptible neoplasms in mammals by administering a therapeutically effective amount of a compound of Formula Ia. The anti-tumor activity of the compounds of Formula Ia may be demonstrated in standard screens commonly used by those skilled in the art for testing compounds with potential anti-tumor activity. These screens are the same screens which are used to demonstrate the anti-tumor activity of commercially available anti-cancer agents such as the vinca alkaloids (see, e.g. Miller et al., in J. Med. Chem. Vol. 20, No. 3-409 (1977) and Sweeney et al. in Cancer Research 38, 2886 (1978)). Suitable testing for the efficacy of the compounds of Formula I are: cytostatic inhibition of the growth of human leukemic cells (CCRF-CEM cell line), animals bearing a tumor system representative of lymphocytic leukemia, 6C 3HED lymphosarcoma, CA-755 adenocarcinoma, P1534J lymphatic leukemia and X5563 plasma cell myeloma.

Of course the use of the compounds of Formula I includes their use in conjunctive therapy with known anti-tumor agents; such conjunctive application facilitating increased efficacy and/or decreased toxicity.

The term "pharmaceutically effective amount" refers to an appropriate amount of a compound of Formula I which is capable of providing chemotherapy to mammals. The anti-tumor compounds are effective over a wide dosage range. For example, dosages per day will normally fall within the range of about 0.1 to about 20 mg/kg of body weight. In the treatment of adult humans, the range of about 0.1 to about 50 mg/kg, in single or divided doses, is preferred. However, it will be understood that the amount of compound actually administered will be determined by a physician, in the light of the relevant circumstances including the condition to be treated, the particular compound to be administered, the chosen route of administration, the age, weight and response of the individual patient, and the severity of the patient's symptoms, and therefore the above dosage ranges are not intended to limit the scope of the invention in any way.

The term "susceptible neoplasm", as defined herein, represents an abnormal growth of tissue in mammals capable of being treated by a compound of Formula I. While the compounds of Formula I are effective against tumors, both solid and non-solid type, the compounds are effective in controlling the growth of rapidly dividing cells because of the compounds cytotoxic nature. It is a special feature of these compounds that they have a broad spectrum of activity, and are accordingly useful against a variety of tumors.

The compounds of the present method are preferably administered as pharmaceutical formulations. These pharmaceutical formulations are comprised of a compound of Formula I in combination with a pharmaceutical carrier, diluent or excipient therefor.

The active ingredient will be present in the formulation in the range of about 1% to about 90% by weight. The active ingredient will usually be mixed with a carrier, or diluted by a carrier, or enclosed with in a carrier which may be in the form of capsule, sachet, paper or other container. When the carrier serves as a diluent, it may be a solid, semi-solid or liquid material which acts as a vehicle, excipient or medium for the active ingredient. Thus, the compositions can be in the form of tablets, pills, powders, lozenges, sachets, cachets, elixirs, suspensions, emulsions, solutions, syrups, aerosols (as a solid or in a liquid medium), ointments containg for example up to 10% by weight of the active compound, soft and hard gelatin capsules, suppositories, sterile injectable solutions and sterile packaged powders.

Some examples of suitable carriers, excipients, and diluents include lactose, dextrose, sucrose, sorbitol, mannitol, starches, gum acacia, calcium silicate, microcrystalline cellulose, polyvinylpyrrolidone, cellulose, water, syrup, methyl cellulose, methyl- and propylhydroxybenzoates, talc, magnesium stearate and mineral oil. The formulations can additionally include lubricating agents, wetting agents, emulsifying and suspending agents, preserving agents, sweetening agents or flavoring agents. The compositions of the invention may be formulated so as to provide quick, sustained release of the active ingredient after administration to the patient by employing procedures well known in the art.

The compositions are preferably formulated in a unit dosage form, each dosage containing from about 5 to about 500 mg, more usually about 25 to about 300 mg, of the active ingredient. The term "unit dosage form" refers to physically discrete units suitable as unitary dosages for human subjects and other mammals, each unit containing a predetermined quantity of active material calculated to produce the desired therapeutic effect, in association with a suitable pharmaceutical carrier.

As is true for large classes of compounds suitable for use as pharmaceuticals, certain sub-groups and certain specific compounds within the generic scope of such inventions are more preferred than others.

## Claims (Claims for the following Contracting State(s): AT, BE, CH, LI, DE, FR, GB, GR, IT, LU, NL, SE)

1. Compounds of the formula 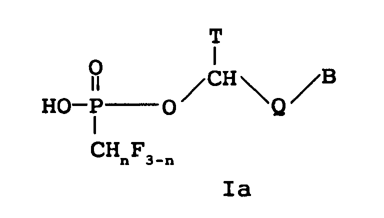 or pharmaceutically acceptable salts thereof, wherein n is an integer zero, one or two, and in which B is a base of the following structural formula : 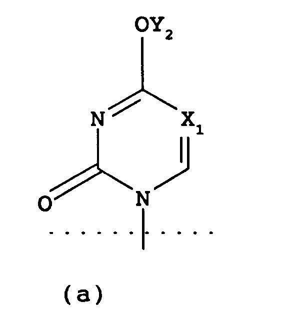 wherein
X₁ is N or CY₁ with Y₁ being H, CH₃, C₂H₅, I, F, Cl, Br, NHR, SR, -C≡CH, -CH=CH₂, or -CH=CHBr, -CH₂CH₂Cl, -CH₂CH₂F, or -CH₂C₆H₄OCH₂C₆H₅ (meta),
with
R being H, CH₃, C₂H₅, CH₂C≡CH, CH₂CH=CH₂ or CH₂CN,
Y₂ is H or C₂H₅, and
Q is a cyclic moiety of the formula 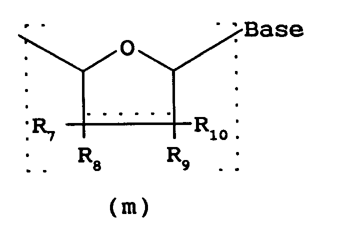
with
R₇ being H, F, Cl
R₈ being H, OH, F, N₃, NH₂, Cl
R₉ being H, OH, F, N₃, NH₂, Cl
R₁₀ being H, F, Cl
T is H, and the dotted line within the structure of formula (m) represents an optional double bond, in which case R₈ and R₉ are omitted and R₇ and R₁₀ are H.

2. A process for preparing a compound having the formula 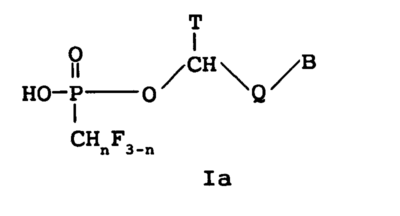 or pharmaceutically acceptable salts thereof, wherein n is an integer zero, one or two, and in which B is a base of the following structural formula; 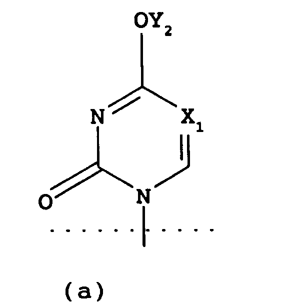 wherein
X₁ is N or CY₁ with Y₁ being H, CH₃, C₂H₅, I, F, Cl, Br, NHR, SR, -C≡CH, -CH=CH₂, or -CH=CHBr, -CH₂CH₂Cl, -CH₂CH₂F, or -CH₂C₆H₄OCH₂C₆H₅ (meta),
with
R being H, CH₃, C₂H₅, CH₂C≡CH, CH₂CH=CH₂ or CH₂CN,
Y₂ is H or C₂H₅, and
Q is a cyclic moiety of the formula 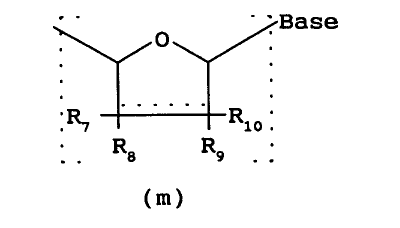
with
R₇ being H, F, Cl
R₈ being H, OH, F, N₃, NH₂, Cl
R₉ being H, OH, F, N₃, NH₂, Cl
R₁₀ being H, F, Cl
T is H, and the dotted line within the structure of formula (m) represents an optional double bond, in which case R₈ and R₉ are omitted and R₇ and R₁₀ are H,
which comprises
(a) coupling of a fluoromethyl phosphonate salt (3) 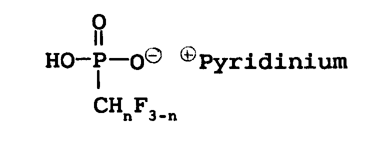 with the appropriate nucleoside (4) 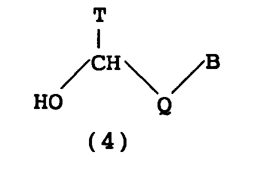 by interaction at about 20°C to 40°C for one to three days in the presence of three to five equivalents of dicyclohexylcarbodiimide (DCC) under anhydrous conditions, preferably in an inert atmosphere ;
(b) isolating and purifying the thus-obtained compound of formula Ia,
(c) optionally converting said compound into its pharmaceutically acceptable salts.

3. Pharmaceutical compositions, containing as active ingredient a compound of formula Ia, in combination with a pharmaceutical carrier, diluent or excipient thereof.

4. Use of the compounds of formula Ia as claimed in claim 1 for the preparation of medicaments useful as antiviral and antitumoral agents.

## Claims (Claims for the following Contracting State(s): ES)

1. A process for preparing a compound having the formula : 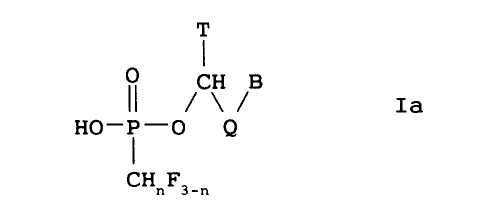 or pharmaceutically acceptable salts thereof, wherein n is an integer zero, one or two, and in which B is a base of the following structural formula : 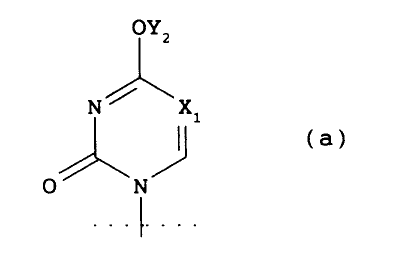 wherein
X₁ is N or CY₁ with Y₁ being H, CH₃, C₂H₅, I, F, Cl, Br, NHR, SR, -C≡CH, -CH=CH₂, or -CH=CHBr, -CH₂CH₂Cl, -CH₂CH₂F, or -CH₂C₆H₄OCH₂C₆H₅ (meta)
with
R being H, CH₃, C₂H₅, CH₂C≡CH, CH₂CH=CH₂ or CH₂CN,
Y₂ is H or C₂H₅, and
Q is a cyclic moiety of the formula 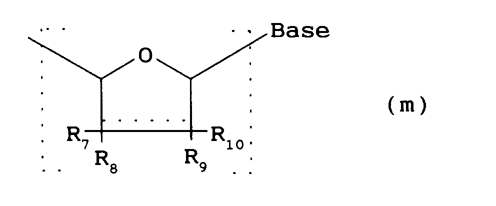
with
R₇ being H, F, Cl
R₈ being H, OH, F, N₃, NH₂, Cl
R₉ being H, OH, F, N₃, NH₂, Cl
R₁₀ being H, F, Cl
T is H, and the dotted line within the structure of formula (m) represents an optional double bond, in which case R₈ and R₉ are omitted and R₇ and R₁₀ are H,
which comprises
(a) coupling of a fluoromethyl phosphonate salt (3) 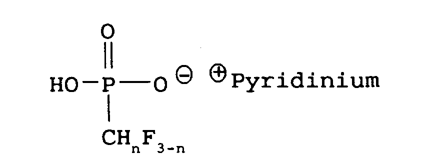 with the appropriate nucleoside (4) 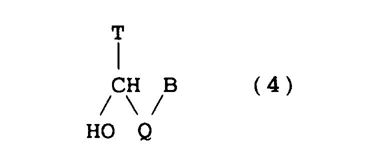 by interaction at about 20°C to 40°C for one to three days in the presence of three to five equivalents of dicyclohexylcarbodiimide (DCC) under anhydrous conditions, preferably in an inert atmosphere ;
(b) isolating and purifying the thus-obtained compound of formula Ia,
(c) optionally converting said compound into its pharmaceutically acceptable salts.

2. Use of the compounds of formula Ia as prepared by the process of claim 1 for the preparation of medicaments useful as antiviral and antitumoral agents.

## Patentansprüche (Patentansprüche für folgende(n) Vertragsstaat(en): AT, BE, CH, LI, DE, FR, GB, GR, IT, LU, NL, SE)

1. Verbindungen der Formel 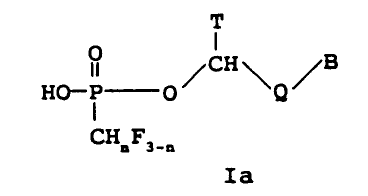 oder pharmazeutisch verträgliche Salze davon, wobei n eine ganze Zahl mit dem Wert null, eins oder zwei ist und B eine Base der folgenden Strukturformel bedeutet 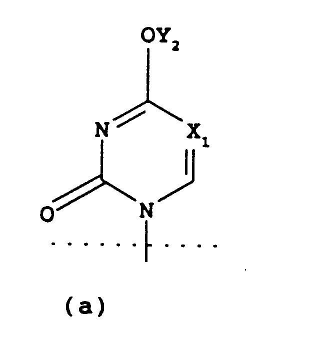 in der
X₁ N oder einen Rest CY₁ bedeutet, wobei Y₁ H, CH₃, C₂H₅, I, F, Cl, Br, NHR, SR, -C≡CH, -CH=CH₂, -CH=CHBr, -CH₂CH₂Cl, -CH₂CH₂F oder -CH₂C₆H₄OCH₂C₆H₅ (meta) darstellt und R H, CH₃, C₂H₅, CH₂C≡CH, CH₂CH=CH₂ oder CH₂CN bedeutet,
Y₂ H oder C₂H₅ bedeutet, und
Q eine cyclische Einheit der Formel 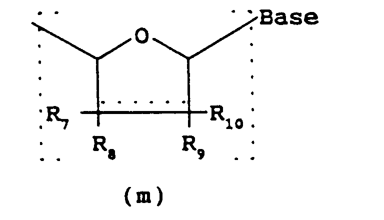
bedeutet, in der
R₇ H, F, Cl bedeutet,
R₈ H, OH, F, N₃, NH₂, Cl bedeutet,
R₉ H, OH, F, N₃, NH₂, Cl bedeutet,
R₁₀ H, F, Cl bedeutet, und
T H bedeutet, und die gestrichelte Linie in der Struktur der Formel (m) eine gegebenenfalls vorhandene Doppelbindung wiedergibt, in diesem Fall fallen R₈ und R₉ weg und R₇ und R₁₀ bedeuten H.

2. Verfahren zur Herstellung einer Verbindung der Formel 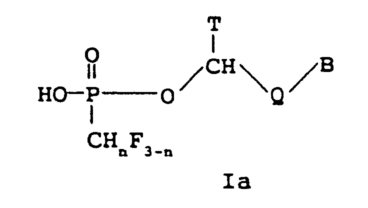 oder pharmazeutisch verträglicher Salze davon, wobei n eine ganze Zahl mit dem Wert null, eins oder zwei ist und B eine Base bedeutet ausgewählt aus der Gruppe mit der folgenden Strukturformel 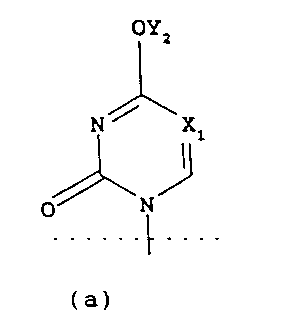 in der
X₁ N oder einen Rest CY₁ bedeutet, wobei Y₁ H, CH₃, C₂H₅, I, F, Cl, Br, NHR, SR, -C≡CH, -CH=CH₂, -CH=CHBr, -CH₂CH₂Cl, -CH₂CH₂F oder -CH₂C₆H₄OCH₂C₆H₅ (meta) darstellt und R H, CH₃, C₂H₅, CH₂C≡CH, CH₂CH=CH₂ oder CH₂CN bedeutet,
Y₂ H oder C₂H₅ bedeutet, und
Q eine cyclische Einheit der Formel 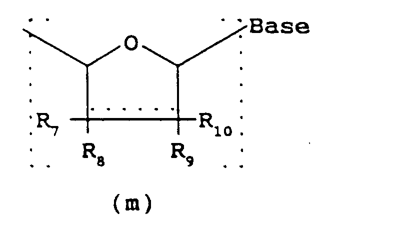
bedeutet, in der
R₇ H, F, Cl bedeutet,
R₈ H, OH, F, N₃, NH₂, Cl bedeutet,
R₉ H, OH, F, N₃, NH₂, Cl bedeutet,
R₁₀ H, F, Cl bedeutet,
T H bedeutet, und die gestrichelte Linie in der Struktur der Formel (m) eine gegebenenfalls vorhandene Doppelbindung wiedergibt, in diesem Fall fallen R₈ und R₉ weg und R₇ und R₁₀ bedeuten H,
umfassend
(a) Kuppeln eines Fluormethylphosphonatsalzes (3) 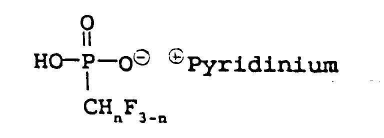 mit dem geeigneten Nucleosid (4) 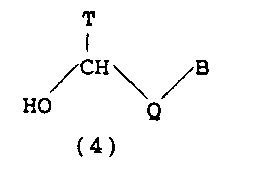 durch Wechselwirkung bei etwa 20°C bis 40°C für ein bis drei Tage in Gegenwart von drei bis fünf Äquivalenten Dicyclohexylcarbodiimid (DCC) unter wasserfreien Bedingungen, vorzugsweise in einer inerten Atmosphäre;
(b) Isolieren und Reinigen der so erhaltenen Verbindung der Formel Ia,
(c) gegebenenfalls Überführen der Verbindung in ihre pharmazeutisch vertraglichen Salze.

3. Arzneimittel, die als Wirkstoff eine Verbindung der Formel Ia in Kombination mit einem pharmazeutischen Träger, Verdünnungsmittel oder Excipienten dafür enthalten.

4. Verwendung der Verbindungen der Formel Ia nach Anspruch 1 zur Herstellung von Arzneimitteln, die als antivirale Mittel und als Antitumormittel geeignet sind.

## Patentansprüche (Patentansprüche für folgende(n) Vertragsstaat(en): ES)

1. Verfahren zur Herstellung einer Verbindung der Formel 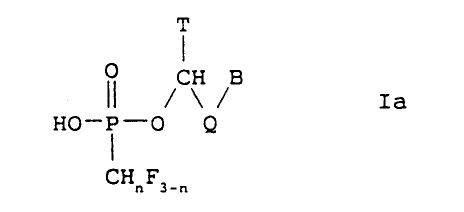 oder pharmazeutisch verträglicher Salze davon, wobei n eine ganze Zahl mit dem Wert null, eins oder zwei ist und B eine Base der folgenden Strukturformel bedeutet 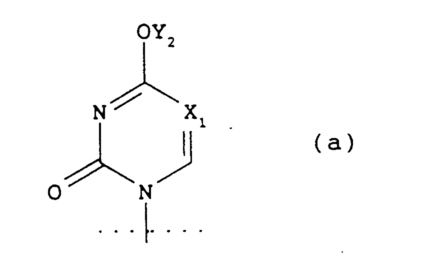 in der
X₁ N oder einen Rest CY₁ bedeutet, wobei Y₁ H, CH₃, C₂H₅, I, F, Cl, Br, NHR, SR, -C≡CH, -CH=CH₂, -CH=CHBr, -CH₂CH₂Cl, -CH₂CH₂F oder -CH₂C₆H₄OCH₂C₆H₅ (meta) darstellt und R H, CH₃, C₂H₅, CH₂C≡CH, CH₂CH=CH₂ oder CH₂CN bedeutet,
Y₂ H oder C₂H₅ bedeutet, und
Q eine cyclische Einheit der Formel 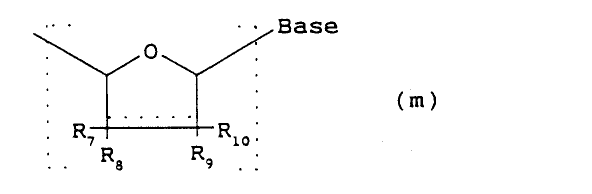
bedeutet, in der
R₇ H, F, Cl bedeutet,
R₈ H, OH, F, N₃, NH₂, Cl bedeutet,
R₉ H, OH, F, N₃, NH₂, Cl bedeutet,
R₁₀ H, F, Cl bedeutet, und
T H bedeutet, und die gestrichelte Linie in der Struktur der Formel (m) eine gegebenenfalls vorhandene Doppelbindung wiedergibt, in diesem Fall fallen R₈ und R₉ weg und R₇ und R₁₀ bedeuten H,
umfassend
(a) Kuppeln eines Fluormethylphosphonatsalzes (3) 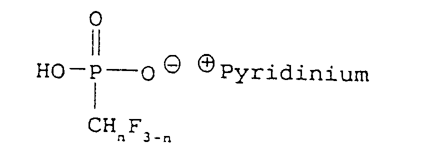 mit dem geeigneten Nucleosid (4) 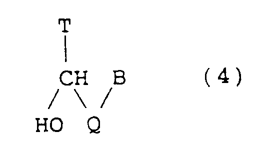 durch Wechselwirkung bei etwa 20°C bis 40°C für ein bis drei Tage in Gegenwart von drei bis fünf Äquivalenten Dicyclohexylcarbodiimid (DCC) unter wasserfreien Bedingungen, vorzugsweise in einer inerten Atmosphäre;
(b) Isolieren und Reinigen der so erhaltenen Verbindung der Formel Ia,
(c) gegebenenfalls Überführen der Verbindung in ihre pharmazeutisch verträglichen Salze.

2. Verwendung der Verbindungen der Formel Ia, wie sie nach dem Verfahren des Anspruchs 1 hergestellt wurden, zur Herstellung von Arzneimitteln, die als antivirales Mittei und als Antitumormittel geeignet sind.

## Revendications (Revendications pour l'(les) Etat(s) contractant(s) suivant(s): AT, BE, CH, LI, DE, FR, GB, GR, IT, LU, NL, SE)

1. Composés de formule: 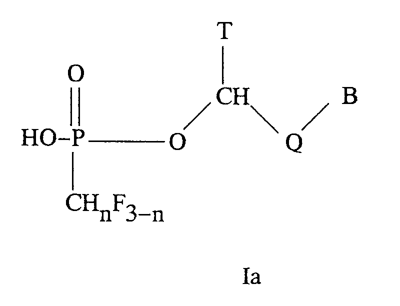 ou sels pharmaceutiquement acceptables de ceux-ci, où n est un entier de 0,1 ou 2, et où B est une base de formule structurale suivante : 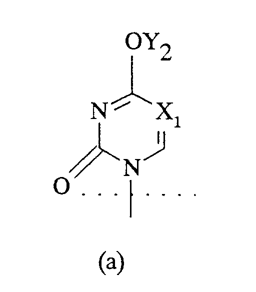 dans laquelle
X₁ est N ou CY₁, Y₁ étant H, CH₃, C₂H₅, I, F, Cl, Br, NHR, SR, -C≡CH, -CH=CH₂ ou -CH=CHBr, -CH₂CH₂Cl, -CH₂CH₂F ou -CH₂C₆H₄OCH₂C₆H₅ (méta),
R étant H, CH₃, C₂H₅, CH₂C≡CH, CH₂CH=CH₂ ou CH₂CN,
Y₂ est H ou C₂H₅, et
Q est une entité cyclique de formule 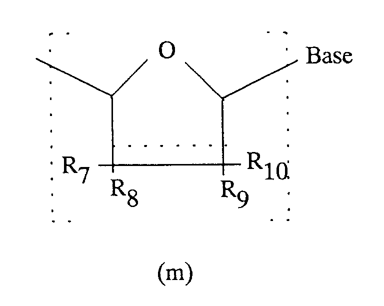
où
R₇ est H, F, Cl
R₈ est H, OH, F, N₃, NH₂, Cl
R₉ est H, OH, F, N₃, NH₂, Cl
R₁₀ est H, F, Cl
T est H, et la ligne en pointillé dans la structure de formule (m) représente une double liaison éventuelle, auquel cas R₈ et R₉ sont omis et R₇ et R₁₀ sont H.

2. Procédé de préparation d'un composé de formule : 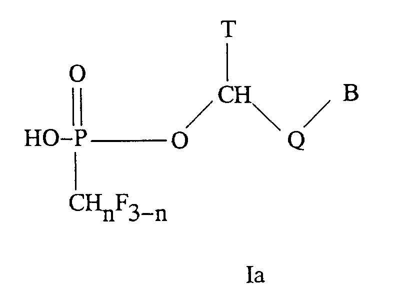 ou de sels pharmaceutiquement acceptables de celui-ci, où n est un entier de 0,1 ou 2, et où B est une base de formule structurale suivante : 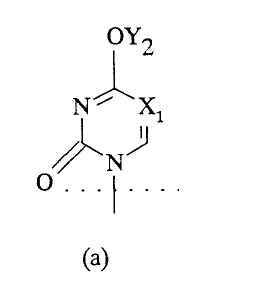 dans laquelle
X₁ est N ou CY₁, Y₁ étant H, CH₃, C₂H₅, I, F, Cl, Br, NHR, SR, -C≡CH, -CH=CH₂ ou -CH=CHBr, -CH₂CH₂Cl, -CH₂CH₂F ou -CH₂C₆H₄OCH₂C₆H₅ (méta),
R étant H, CH₃, C₂H₅, CH₂C≡CH, CH₂CH=CH₂ ou CH₂CN,
Y₂ est H ou C₂H₅, et
Q est une entité cyclique de formule 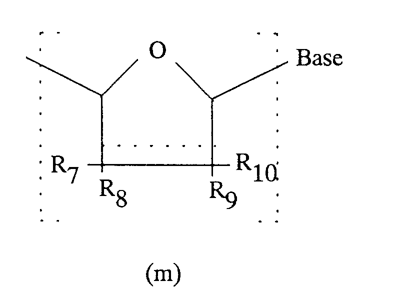
où
R₇ est H, F, Cl
R₈ est H, OH, F, N₃, NH₂, Cl
R₉ est H, OH, F, N₃, NH₂, Cl
R₁₀ est H, F, Cl
T est H, et la ligne en pointillé dans la structure de formule (m) représente une double liaison éventuelle, auquel cas R₈ et R₉ sont omis et R₇ et R₁₀ sont H,
qui comprend
(a) le couplage d'un sel fluorométhylphosphonate (3) 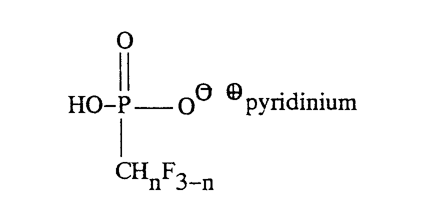 avec le nucléoside (4) approprié 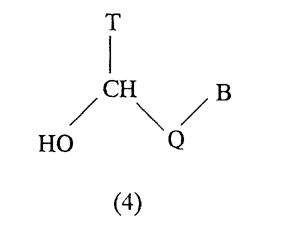 par interaction à environ 20°C à 40°C pendant un à trois jours en présence de trois à cinq équivalents de dicyclohexylcarbodiimide (DCC) dans des conditions anhydres, de préférence dans une atmosphère inerte;
(b) l'isolement et la purification du composé de formule Ia ainsi obtenu,
(c) la conversion éventuelle dudit composé en ses sels pharmaceutiquement acceptables.

3. Compositions pharmaceutiques contenant comme ingrédient actif un composé de formule Ia en combinaison avec un support, diluant ou excipient pharmaceutique pour celui-ci.

4. Utilisation des composés de formule Ia selon la revendication 1 pour la préparation de médicaments utiles comme agents antiviraux et antitumoraux.

## Revendications (Revendications pour l'(les) Etat(s) contractant(s) suivant(s): ES)

1. Procédé de préparation d'un composé de formule : 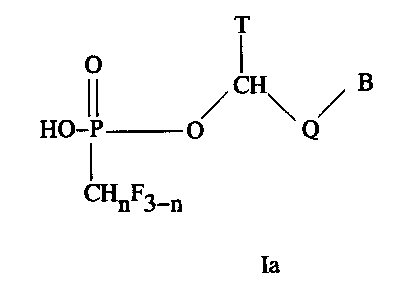 ou de sels pharmaceutiquement acceptables de celui-ci, où n est un entier de 0,1 ou 2, et où B est une base de formule structurale suivante : 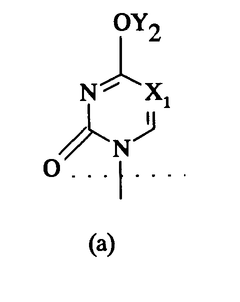 dans laquelle
X₁ est N ou CY₁, Y₁ étant H, CH₃, C₂H₅, I, F, Cl, Br, NHR, SR, -C≡CH, -CH=CH₂ ou -CH=CHBr, -CH₂CH₂Cl, -CH₂CH₂F ou -CH₂C₆H₄OCH₂C₆H₅ (méta),
R étant H, CH₃, C₂H₅, CH₂C≡CH, CH₂CH=CH₂ ou CH₂CN,
Y₂ est H ou C₂H₅, et
Q est une entité cyclique de formule 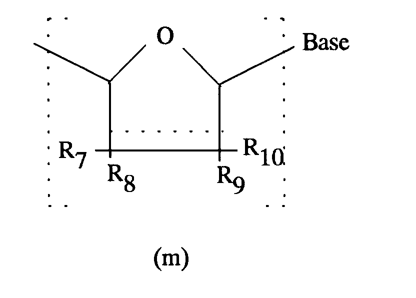
où
R₇ est H, F, Cl
R₈ est H, OH, F, N₃, NH₂, Cl
R₉ est H, OH, F, N₃, NH₂, Cl
R₁₀ est H, F, Cl
T est H, et la ligne en pointillé dans la structure de formule (m) représente une double liaison éventuelle, auquel cas R₈ et R₉ sont omis et R₇ et R₁₀ sont H,
qui comprend
(a) le couplage d'un sel fluorométhylphosphonate (3) 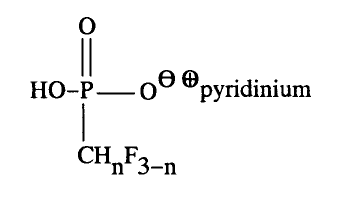 avec le nucléoside (4) approprié 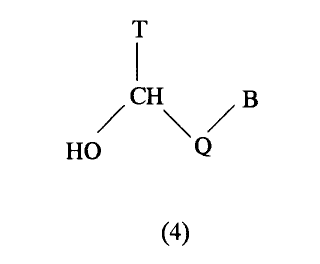 par interaction à environ 20°C à 40°C pendant un à trois jours en présence de trois à cinq équivalents de dicyclohexylcarbodiimide (DCC) dans des conditions anhydres, de préférence dans une atmosphère inerte ;
(b) l'isolement et la purification du composé de formule la ainsi obtenu,
(c) la conversion éventuelle dudit composé en ses sels pharmaceutiquement acceptables.

2. Utilisation des composés de formule Ia tels que préparés par le procédé selon la revendication 1 pour la préparation de médicaments utiles comme agents antiviraux et antitumoraux.
